# EUROPEAN PATENT SPECIFICATION

(11) **EP 2 938 318 B1**
(45) Date of publication and mention of the grant of the patent: **04.09.2019**
(21) Application number: 13795447.5
(22) Date of filing: 15.11.2013
(51) Int. Cl.: A61K 8/26, A61K 8/41, A61Q 11/00, A61K 8/02

(54) **ORAL CARE COMPOSITION HAVING AN ADDUCT OF CLAY AND ANTIBACTERIAL AGENT**
MUNDPFLEGEZUSAMMENSETZUNG MIT EINEM ADDUKT AUS TON UND ANTIBAKTERIELLEM WIRKSTOFF
COMPOSITION DE SOINS BUCCAUX DOTÉE DU COMPOSÉ D'ADDITION D'UNE ARGILE ET D'UN AGENT ANTIBACTÉRIEN

(30) Priority: 27.12.2012 EP 12199421
(43) Date of publication of application: 04.11.2015
(73) Proprietor: Unilever N.V., 3013 AL Rotterdam (NL); Unilever PLC, London, Greater London EC4Y 0DY (GB)
(72) Inventor: BHATTACHARYA, Arpita, Bangalore 560 066 (IN); CHANDRASEKARAN, Sembian, Mumbai 400 099 (IN); GHOSH DASTIDAR, Sudipta, Bangalore 560 066 (IN); GOEL, Satish Kumar, Mumbai 400 099 (IN); IYER, Meenakshi, Mumbai 400 099 (IN); SAJI, Maya Treesa, Bangalore 560 066 (IN)
(74) Representative: Tansley, Sally Elizabeth
(86) International application number: PCT/EP2013/073982
(87) International publication number: WO 2014/102032

(56) References cited:
- GB-A- 710 129
- JP-A- 2001 122 749
- JP-A- 2004 359 483
- US-B1- 6 649 147

## Description

### FIELD OF THE INVENTION

The invention relates to toothpaste compositions having antibacterial activity.

### BACKGROUND OF THE INVENTION

Most oral care compositions such as toothpastes usually contain one or more antibacterial agent for oral hygiene. Triclosan is an antibacterial agent which has found widespread acceptance. Zinc chloride, zinc citrate and other zinc salts represent another class of widely used antibacterial agents.

Triclosan is efficacious over a wide spectrum. Currently it is used in many products but there are concerns about its effects on human health. It is believed that triclosan may be banned or restricted in the near future. Many other antimicrobial agents have been suggested for use in personal care products. However in the case of oral care products, more stringent safety regulations limit the antibacterial agents that can be used. Some antibacterial agents are incompatible with common abrasives such as chalk and also with surfactants.

Therefore industry needs newer antibacterial agents for oral care products.

Cationic agents like chlorhexidine and cetyl pyridinium chloride are believed to be more substantive and can be readily absorbed and gradually released. However, the successful formulation of a cationic antibacterial agent into marketable toothpaste has not yet been achieved and this is at least in part due to incompatibility of cationic antibacterial agents with some of the common ingredients found in toothpaste.

Nevertheless, some attempts can be found in published documents.

WO2007/063506A1 (P&G) discloses a dentifrice composition having a hydrophilic clay material, a phytic acid compound, an oral care active and a polar solvent carrier wherein the composition is substantially free of abrasive material. The novelty of this disclosure lies in minimizing the use of abrasives.

WO2003/002056 (Church And Dwight Co Inc) discloses an oral composition having antibacterial effective amount of cetylpyridinium chloride and guar hydroxypropyltrimonium chloride to bind to compounds which undesirably bind to cetylpyridinium chloride thereby enabling the cetylpyridinium chloride to effectively bind to tooth surfaces and perform an antibacterial function.

A bipolar clay based antimicrobial particle has been disclosed in US2012/0177712A1 (Unilever). The precursor of the particle is an asymmetric 1:1 or 2:1:1 clay particle comprising alternating tetrahedral and octahedral sheets terminating with a tetrahedral sheet at one external surface plane and an octahedral sheet at another external surface plane with an antimicrobial group attached to the coordinating cation on one of the said external surface plane selected from a quaternary ammonium material comprising a single alkyl or alkenyl long chain having an average chain length greater than or equal to C₂₀, or a quaternary ammonium material.

Further, it is disclosed that the bipolar antimicrobial particle can be used in diverse applications including oral care compositions. However toothpastes have alkaline pH. Therefore, complexes, adducts, encapsulates and other types of formulations and compositions of antibacterial agents which are otherwise stable under ambient conditions generally tend to lose stability and/or efficacy. Prolonged efficacy is an important feature of any toothpaste claiming antibacterial action.

JP2004359483A (TOPY IND) discloses a modified fluorine silicate powder which having thickening and emulsifying action and in which the quantity of fluorine ion eluted in a hot water elution test is decreased. The solution lies by modifying a fluorine-containing swellable layered silicate used as a host mineral with an organic ammonium cation modifier having a hydrophobic group and a metallic ion exchanger containing a metallic ion exchangeable for Li or Na among the host layers. It is suitable that the organic ammonium cation in the powder is greater than or equal to 10 equiv % to the cation exchange capacity of the fluorine-containing swellable silicate of the host and the metallic ion in the powder is greater than or equal to 10 equiv. % to the cation exchange capacity of the fluorine-containing swellable silicate of the host.

JP 2001 122749 A (KISHO KK) discloses use of smectite as an abrasive for teeth to overcome the defects of the conventional dental abrasives which generally gives the teeth and the gingival tissues damages or irritates the oral mucous membranes.

GB710129A (AMM I DENT INC, 1954) discloses a dental paste having an antibiotic such as penicillin, a polishing agent; a surfactant and an organophyllic gelling agent in which the antibiotic, polishing agent and detergent are dispersed in the vehicle. The preferred gelling agents are bentonite and its derivative, e.g. called Bentone and aluminium stearate.

US6569408B (P&G, 2003) discloses a composition for delivering an oral care substance to the oral cavity, comprising an organosiloxane resin, a volatile carrier capable of solubilizing the organosiloxane resin, rheology modifier; and at least one oral care substance.

GB 710 129 discloses a dental paste having an antibiotic, a polishing agent (calcium carbonate) and an anionic surfactant. The antibiotic is said to be stable and maintains its potency for long periods of time.

We have now determined that toothpaste containing the bipolar antimicrobial particle provides instantaneous antibacterial activity which also surprisingly lasts significantly longer than conventional compositions thereby providing significant protection against bacterial re-growth, a phenomenon commonly observed with some of the marketed antibacterial toothpastes.

### SUMMARY OF THE INVENTION

According to a first aspect is disclosed a toothpaste composition having:
(i) 5 to 60 wt% abrasive wherein said abrasive is a calcium containing abrasive;
(ii) an anionic surfactant; and
(iii) a bipolar antimicrobial particle whose precursor is an asymmetric 1:1 or 2:1:1 clay particle comprising alternating tetrahedral and octahedral sheets terminating with a tetrahedral sheet at one external surface plane and an octahedral sheet at another external surface plane with an antimicrobial agent attached to the coordinating cation on one of the said external surface plane where the antibacterial agent is a quaternary ammonium material.

Preferably the median diameter of the antibacterial particles (D50) is 0.1 to 10 µm.

### DETAILED DESCRIPTION

The composition has 5 to 60 wt% abrasive selected from calcium based abrasive or silica based abrasive, an anionic surfactant and the bipolar antimicrobial particle.

### Abrasive

A toothpaste generally contains an abrasive. Gels usually contain silica, whereas opaque creams generally contain calcium based abrasives, especially chalk. The quantity of the abrasive needs some control because excess abrasive causes more abrasion. Further, uncontrolled amount of abrasives will also adversely affect viscosity of the paste.

Preferred toothpaste compositions have 25 to 60 wt% abrasive, more preferably 30 to 50 wt% and most preferably 45 to 55 wt% abrasive which is silica based or calcium based.

Toothpaste compositions will have calcium containing abrasive. A particularly preferred abrasive is fine ground natural chalk (FGNC). It is obtained from limestone or marble. FGNC may also be modified chemically or physically by coating during or after milling by heat treatment. Typical coating materials include magnesium stearate and oleate. The morphology of FGNC may also be modified during the milling process by using different milling techniques, for example, ball milling, air-classifier milling or spiral jet milling. FGNC can be used as the sole calcium containing abrasive. However, FGNC can also be used with other calcium containing abrasives to balance the abrasion.

Other preferred calcium containing abrasives include dicalcium phosphate (DCP), calcium pyrophosphate and precipitated calcium carbonate (PCC). When a combination of Calcium containing abrasives is used, it is preferred that FGNC is 35 to 100 %, more preferably 75 to 100 % and especially from 95 to 100 % of the total abrasive. In such cases, the balance, most preferably, is PCC.

Other abrasives can also be used depending upon the intended degree of abrasion and the composition of the paste. These include synthetic abrasive polishing agents such as amorphous precipitated silica and silica gels. Other abrasives include magnesium carbonate, sodium metaphosphate, potassium metaphosphate, zirconium silicate, potassium metaphosphate, magnesium orthophosphate, tricalcium phosphate, magnesium orthophosphate, trimagnesium phosphate, aluminum silicate, zirconium silicate and perlite.

### Surfactants

Toothpastes generally contain a surfactant, also commonly referred to as sudsing agent. Suitable surfactants are those which are reasonably stable and provide foam throughout a wider pH range. The surfactant is anionic. Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate and sodium coconut monoglyceride sulfonates are examples of anionic surfactants of this type.

Preferred compositions contain 0.25 to 12 wt%, more preferably from 0.5 to 8 wt%, and most preferably from 1 to about 6 wt% anionic surfactants.

Some anionic surfactants, in particular, sodium lauryl sulphate itself have antibacterial effect. Such action provides some degree of instant antibacterial effect. However, this effect is generally very short-lived. Therefore, the more sustainable antibacterial action is provided by the antibacterial agent such as triclosan. Some of the tests which are routinely performed to quantify the antibacterial action show inconsistent results because the procedure does not distinguish between the effect of the anionic surfactant and that of the antibacterial action. However, single species assay can provide more accurate results because the test concentrates only on the effect of the antibacterial agent.

Other surfactants like nonionic, amphoteric or zwitterionic surfactants may also be included.

Nonionic surfactants can be broadly defined as compounds produced by the condensation of alkylene oxide groups (hydrophilic in nature) with an organic hydrophobic compound which may be aliphatic or alkyl- aromatic in nature. Examples of suitable nonionic surfactants include poloxamers (sold under trade name PLURONIC®), polyoxyethylene, polyoxyethylene sorbitan esters (sold under trade name TWEENS®), POLYOXYL® 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and mixtures of such materials.

Useful amphoteric surfactants can be broadly described as derivatives of aliphatic secondary and tertiary amines in which the aliphatic radical is a straight chain or branched and wherein one of the aliphatic substituent contains from about 8 to about 18 carbon atoms and one contains an anionic water-solubilizing group, e.g., carboxylate, sulfonate, sulfate, phosphate, or phosphonate. Other suitable amphoteric surfactants are betaines, specifically cocamidopropyl betaine. Mixtures of amphoteric surfactants can also be used.

### The bipolar antimicrobial particle

While most antibacterial toothpastes provide immediate antibacterial effect, which in many cases is provided by the anionic surfactant; this effect wears off very rapidly. When this happens, the residual bacterial cells multiply. This is also known as bacterial re-growth and it is usually studied by bacterial re-growth assay.

While in US2012/0177712A1 (Unilever), it was disclosed that the antibacterial particle is suitable for variety of applications, we now find that the particle used in oral care compositions also provides significantly longer lasting efficacy.

It is particularly preferred that the median particle size (D50) of this particle is 0.1 to 10 µm, more preferably 0.4 to 1 µm and most preferably 0.5 to 0.8 µm.

When the particles are of an increased size, the available surface area is believed to be lower. Accordingly, as the preferred particles have significantly lower size, is believed that more area is available for the antibacterial agent to form adduct therewith. Without further being bound by theory, it is believed that this makes more effective use of the antibacterial agent, thereby providing opportunity for substantial reduction in the dosage of the antibacterial agent. While conventional antibacterial agents need an optimised dosage of 0.2 to 0.5 wt%, the antibacterial agent, delivered in the form of the disclosed bipolar antibacterial particle, is seen to be highly effective even at an (actual) dosage of 0.01 %.

While not wishing to be further bound by theory it is believed that the preferred particles are appropriately sized to fit inside the interstitial spaces on the surface of the teeth which is believed to be a reason for prolonged efficacy. It is further believed that particles of lower size reduce, to a significant extent, any unfavorable interaction between the anionic surfactant and antibacterial agent even when the particle contains e.g., cetyl pyridinium chloride which is a quaternary ammonium compound and this is also believed to be a cause for longer lasting antibacterial benefits.

Particles of lower size also provide an effective mechanism for an increased loading of the antibacterial agent, if so desired.

Particle size distribution (D50) is also known as the median diameter or the medium value of the particle size distribution, it is the value of the particle diameter at 50 % in the cumulative distribution. It is an important parameter characterizing particle size. For example, if D50=5.8 µm, then 50 % of the particles in the sample are larger than 5.8 µm, and 50 % smaller than 5.8 µm. D50 is usually used to represent the particle size of group of particles. The D50 is the size in microns that splits the distribution with half above and half below this diameter.

The precursor of the antibacterial particle with bipolar topospecific characteristics is an asymmetric 1:1 or 2:1:1 clay particle having alternating tetrahedral and an octahedral sheets terminating with a tetrahedral and an octahedral sheet at exterior surface planes. Particle of 1:1 clay is particularly preferred as the precursor. Preferred 1:1 clays include kaolinite and serpentine subgroups of minerals. The species included within the kaolinite subgroup include but are not limited to kaolinite, dickite, halloysite and nacrite. The species within the serpentine subgroup include but are not limited to chrysolite, lizardite, and amesite. Preferred 2:1:1clays include chlorite group of minerals. The chlorite comprises tetrahedral-octahedral-tetrahedral sheets like 2:1 clays, with an extra weakly bound brucite like layerbetween tetrahedral layers. The tetrahedral sheet preferably comprises coordinating tetrahedral cations of silicon. The tetrahedral sheet may also include isomorphously substituted coordinating tetrahedral cations which are not silicon. Isomorphously substituted coordinating tetrahedral cations include, but are not limited to, cations of aluminum, iron or boron.

The octahedral sheet preferably has coordinating octahedral cation of aluminum. The octahedral sheet may also comprise isomorphously substituted coordinating octahedral cations which are not aluminium. Isomorphously substituted coordinating octahedral cations include cations of magnesium or iron. It is preferred that the antimicrobial agent is attached to the coordinating cations on the exterior side of one of the external surface planes. Accordingly, the antimicrobial agent is attached to coordinating cations on the exterior side of the tetrahedral sheet. Alternatively, the antimicrobial molecule is attached to the coordinating cations on the exterior side of the octahedral sheet. Coordinating cations on the exterior side of each of the tetrahedral and the octahedral surface sheets are attached to a antimicrobial molecule, with the proviso that the antimicrobial molecule attached to the coordinating cations on the exterior side of the tetrahedral surface sheet is not identical to the molecule attached to the coordinating cations on the exterior side of the octahedral surface sheet. The antimicrobial agent is preferably attached to the coordinating cations on the external surface of the octahedral surface plane and is not preferably attached to coordination cations of non-exterior tetrahedral or octahedral plane or on the interior side of the surface sheets.

In the above mentioned particulate antimicrobial the clay:antibacterial ratio is from 1:0.001 to 1:1 more preferably from 1:0.001 and 1:0.1.

It is preferred that in the antimicrobial particle, the antibacterial agent is attached to coordinating cations on the external surface of the octahedral surface plane.

Preferred oral care compositions contain 0.1 to 10 wt% bipolar antimicrobial particles, more preferably 0.5 to 5 wt% particles. It is further preferred that such particle contains 1 % to 60 % loading of the antimicrobial agent. A particularly preferred antibacterial agent is Cetylpyridinium chloride.

Further details of the particle can be found in WO2011/036031A1 (Unilever).

The antibacterial agent is a quaternary ammonium material. Preferably the quaternary ammonium material is selected from a quaternary ammonium material comprising a single alkyl or alkenyl long chain having an average chain length greater than or equal to C₂₀ or a quaternary ammonium material selected from cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride, domiphen bromide.

More preferarbly the quaternary ammonium material is selected from cetylpyridinium chloride (CPC), cetyltrimethylammonium chloride (CTAC), cetyltrimethylammonium bromide (CTAB), benzalkonium chloride (BKC), benzethonium chloride, cetrimide or quaternium.

Cetylpyridinium chloride (CPC) is particularly preferred. It is well known as an antibacterial agent especially for the inhibition of plaque.

The antibacterial activity of cetylpyridinium chloride is, without being bound to the theory, believed to be linked to the cationic charge of its amine group. Thus, cetylpyridinium chloride is attracted to and binds to negatively-charged protein moieties on the cell membrane or cell wall of the microorganism and to tooth surfaces which are also typically negatively charged. The resulting attachment to microorganisms disrupts the cell wall structure causing leakage of the intracellular fluids, eventually killing the associated microorganism. However, cetylpyridinium chloride is generally not effective in many systems because of its tendency to complex with component that carry a negative charge. When bound to the clay in this manner, cetylpyridinium chloride is unavailable for effective contact with tooth surfaces and microorganisms, thereby rendering the active agent ineffective for its intended purpose.

For this reason, cetylpyridinium chloride has not been totally effective in typical oral care products for the treatment and/or prevention of gingivitis, plaque, periodontal disease, and/or breath malodor. For example, toothpaste compositions typically include anionic surfactants and artificial sweetening agents. These components of toothpaste compositions typically bind to cetylpyridinium chloride and thereby render it ineffective or substantially less effective as an antibacterial agent. Other components typically found in a toothpaste composition such as abrasives also bind to cetylpyridinium chloride. Accordingly, the use of cetylpyridinium chloride in toothpaste compositions has been problematic. Even in commercial mouthwash products that contain cetylpyridinium chloride, the availability of cetylpyridinium chloride at tooth surfaces is very low and therefore its antibacterial effectiveness is limited.

### Preferred ingredients

In addition to the ingredients which have already been described, the toothpaste preferably includes one or more of the preferred ingredients which will now be described.

### Smectite clay

Preferred compositions include smectite clay which is in addition to the clay present by way of the antibacterial particle.

Smectites constitute a group in the class of natural aluminosilicate minerals known as phyllosilicates or layered silicates. Preferred smectite clay is selected from montmorillonites (bentonites, hectorites and derivatives thereof); purified aluminium magnesium silicates (various grades are commercially available as VEEGUM® from R. T. Vanderbilt Company); purified sodium magnesium silicates (commercially available as LAPONITE® in various grades); organically modified smectites including tetra alkyl and/or trialkyl ammonium smectites (organically modified montmorillonite clays) such as quaternium-18 bentonite, quaternium-18 hectorite, stearalkonium bentonite and stearalkonium hectorite/ and mixtures thereof. Aluminium magnesium silicate clays are particularly preferred. An example is VEEGUM® HV. The clay tends to swell when exposed to water. Preferred toothpaste compositions contain 0.2 to 3 wt% clay. More preferred compositions include 0.5 to 1 wt% clay.

The smectite clay not only plays a role in sensory profile as is believed, but it also plays a role in thickening the composition, as the reduced content of thickening silica otherwise leads to a product with lower viscosity.

### Thickening silica

The disclosed composition contain 0 to 2 wt% thickening silica, which implies that compositions may or may not contain thickening silica and this is particularly the case where the composition contains Smectite clay. Whenever the smectite clay is present, its (silica's) upper limit is 2 wt%. Conventional gel toothpastes generally contain upto 8.5 wt% thickening silica whereas opaque toothpastes typically contain 4 to 10 wt%. The toothpaste compositions can include up to 2 wt% thickening silica. Preferred compositions have 1.5 wt%, or even less than 1 wt% thickening silica. Optimal compositions have less than 0.5 wt% thickening silica. Highly preferred compositions do not contain thickening silica.

When present, preferred thickening silicas include AEROSIL®T series from Degussa or the CAB-O-SIL® series from Cabot Corporation, silica gels such as the SYLODENT® or SYLOX® series from W. R.Grace & Co or precipitated silica such as ZEOTHIX® 265 from J. M. Huber Corporation. Useful silica thickeners also include ZEODENT® 165, ZEODENT® 163 and/or 167 and ZEOFREE® 153, 177, and/or 265 silicas, all available from J. M. Huber Corporation. Other preferred thickening silicas include MFIL®, MFIL®-P (From Madhu Silica), SIDENT® 22 S and AEROSIL® 200 (Ex. Evonik Industries), SYLODENT® and PERKASIL® thickening silicas from WR Grace & Company and Tixosil® 43 and 331 from Rhodia, synthetic finely divided pyrogenic silica such as those sold under the trademarks SYLOID® 244, SYLOID® 266 and AEROSIL® D-200.

### Humectant

Humectants are generally included in toothpastes for a soft a supple mouth feel. Humectants also reduce the tendency of toothpastes to lose moisture. Preferred toothpaste compositions contain 3.5 to 40 wt% humectants. Further preferred compositions have 10 to 40 wt%, more particularly 10 to 20 wt% humectants.

A particularly preferred humectant is sorbitol, generally available as 70% aqueous solution.

Other preferred humectants include glycerine, maltitol and xylitol. More preferred toothpastes contain glycerine and sorbitol for a lubricated mouth feel, but their cumulative levels should not exceed the disclosed upper limit. Lower humectant content provides an effective way to reduce the cost of the product.

### Binder

Preferred toothpaste compositions have a binder, which lends a good structure to the paste. Cellulosic binders are especially preferred. Preferred cellulosic binders include cellulose ethers, which include hydroxyethyl cellulose (HEC), hydroxypropyl cellulose (HPC), ethylhydroxyethyl cellulose (EHEC), carboxymethyl cellulose (CMC), carboxymethylhydroxyethyl cellulose (CMHEC), hydroxypropylhydroxyethyl cellulose (HPHEC), methyl cellulose (MC), methylhydroxypropyl cellulose (MHPC), methylhydroxyethyl cellulose (MHEC), carboxymethyl methyl cellulose (CMMC), hydrophobically modified carboxymethyl cellulose (HMCMC), hydrophobically modified hydroxyethyl cellulose (HMHEC), hydrophobically modified hydroxypropyl cellulose (HMHPC), hydrophobically modified ethylhydroxyethyl cellulose (HMEHEC), hydrophobically modified carboxymethylhydroxyethyl cellulose (HMCMHEC), hydrophobically modified hydroxypropylhydroxyethyl cellulose (HMHPHEC), hydrophobically modified methyl cellulose (HMMC), hydrophobically modified methylhydroxypropyl cellulose (HMMHPC), hydrophobically modified methylhydroxyethyl cellulose (HMMHEC), and hydrophobically modified carboxymethylmethyl cellulose (HMCMMC).

Other cellulosic binders include cationic hydroxyethyl cellulose (cationic HEC), cationic hydrophobically modified hydroxyethyl cellulose (cationic HMHEC) and microcrystalline cellulose.

A highly preferred binder is Sodium carboxymethyl cellulose (SCMC). Particularly preferred sodium carboxymethyl celluloses include those with degree of substitution of from 0.6 to 0.99, preferably from 0.7 to 0.95.

In addition to, or as a replacement of cellulosic binder, guar gum or its derivatives could be used, however, such binders are less preferred to the cellulosic ones. Such derivatives include carboxymethyl guar (CM guar), hydroxyethyl guar (HE guar), hydroxypropyl guar (HP guar), carboxymethylhydroxypropyl guar (CMHP guar), cationic guar, hydrophobically modified guar (HM guar), hydrophobically modified carboxymethyl guar (HMCM guar), hydrophobically modified hydroxyethyl guar (HMHE guar), hydrophobically modified hydroxypropyl guar (HMHP guar), cationic hydrophobically modified hydroxypropyl guar (cationic HMHP guar), hydrophobically modified carboxymethylhydroxypropyl guar (HMCMHP guar) and hydrophobically modified cationic guar (HM cationic guar).

Other less preferred gums include xanthan gum, carrageenan and derivatives, such as Irish moss and viscarin, gellan gum, sclerotium gum and derivatives, pullulan, rhamsan gum, welan gum, konjac, curdlan, algin, alginic acid, alginates and derivatives, starch phosphate derivatives, agar and derivatives, gum arabic and derivatives, pectin and derivatives, chitosan and derivatives, karaya gum, locust bean gum, natto gum, tragacanth gum, chitin derivatives, gelatin, betaglucan, dextrin, dextran, cyclodextrin and polyquaterniums, furcellaren gum, ghatti gum, psyllium gum, quince gum, tamarind gum, larch gum, and tara gum.

In the case of preferred compositions which have cellulosic binder in addition to the smectite clay, the ratio of the amount of smectite clay to the cellulosic binder is in the range of 1:0.2 to 1:0.9. Excessive cellulosic binder leads to compositions which show a tailing effect while dispensing the composition on a toothbrush, which is an undesirable in-use effect. On the other hand, most of the cellulosic binders and some others like carrageenan are expensive ingredients. Therefore, although their use can restore the viscosity of the product, it would not be an economically viable option.

### Optional ingredients

In addition to the ingredients described earlier, preferred compositions can include one or more other ingredients as described herein after.

### Other thickening agents

Preferred toothpaste compositions may also include one or more other thickening agents such as carboxyvinyl polymers which include carbomers which are commercially available from B. F. Goodrich as the CARBOPOL® series, including CARBOPOL® 934, 940, 941 and 956.

Other preferred grades include acrylates/C₁₀-₃₀ alkyl acrylate crosspolymers which are commercially available as ULTREZ® 21, PEMULEN® TR-1, and PEMULEN® TR-2, from Noveon Corporation. Preferred compositions can include 0.05 to 10 wt%, more preferably 0.1 to 5 wt%, and even more preferably 0.25 to about 4 wt% of other thickening agents.

### De-sensitising agents

A de-sensitising agent is a potassium salt selected from potassium nitrate, potassium chloride, potassium citrate, potassium tartarate and potassium acetate used preferably from 0.5 to 3 wt%, more preferably from 1 to 2.5 wt % and especially from 1.7 to 2.2 wt%.

### Silicates

Preferred toothpaste compositions can have alkali metal silicate. The alkali metal is sodium or potassium, preferably sodium. Sodium silicate is generally available as 10 to 40 % aqueous solution, most common being 30 % solution. Sodium silicate is available as neutral sodium silicate or alkaline sodium silicate. Preferred toothpastes have neutral sodium silicate. Sodium silicate is available with varying ratios of Na₂O: SiO₂.

Sodium silicate with Na₂O: SiO₂ ratio in the range of 3.0 to 3.8 is preferred, more highly preferred range being 3.25 to 3.5. Preferred toothpastes include 0.1 to 5 wt% silicate (on dry weight basis). Thus, a 30 % solution of sodium silicate is added to the composition in an amount in the range of 0.3 to 16 wt%.

### Anti-caries agent

Preferred compositions can include one or more anti-caries agent. Such agents are typically fluorides. It is preferred that the source of fluoride is an alkali-metal salt of monofluorophosphoric acid, preferably sodium monofluorophosphate (SMFP).

SMFP is the fluoride source of choice when it comes to toothpaste compositions having Calcium based abrasives, especially chalk as sodium fluoride reacts with the calcium carbonate to form insoluble calcium fluoride which has limited anti-caries activity. Preferred compositions include 0.01 to 2 wt%, more preferably 0.15 to 1 wt% and especially preferably 0.2 to 0.5 wt% anti-caries agent. It is preferable to maintain the free fluoride ion concentration from 100 to 2000 ppm, preferably from 900 to 1500 ppm. Other preferred anti-caries agents include sodium- and stannous fluoride, aminefluorides, sodium trimetaphosphate and casein.

### Other antibacterial agents

Additional anti-bacterial agents can be present in the composition, though not strictly necessary. Examples include triclosan and other halogenated bisphenolic compounds such as 2,2' methylenebis-(4-chloro-6-bromophenol).

### Preservatives

Toothpastes with calcium containing abrasives especially chalk are prone to bacterial growth. Certain preservatives, e.g. methyl, ethyl, butyl, propyl and isopropyl esters of parahydroxybenzoic acid may be particularly useful against bacterial growth. A mixture of methyl, ethyl, butyl and propyl esters of parahydroxybenzoic acid is particularly preferred.

The activity of this mixture can be enhanced by adding phenoxyethanol. Formaldehyde and dimethyl hydantoin are other preferred preservatives. Preservatives are generally included at 0.05 to 0.8 wt%.

### Antioxidants

Preferred antioxidants are those which are compatible with other components and are not hazardous to health. These include ascorbic acid, ascorbyl palmitate, thiodipropionic acid, calcium ascorbate, dilauryldithiopropionate, gum guaiac, sodium ascorbate, butylated hydroxyl toluene, butylated hydroxyl anisole, and tocopherols. Mixture of antioxidants can be used.

When present, the antioxidant is added in a level effective to reduce or mitigate discoloration that would otherwise result from oxidation of the components of the toothpastes. Preferred levels are from 0.01 to 1 wt%.

### Sweetening agents

Toothpastes may also contain a sweetening agent. Preferred sweetening agents include sodium saccharin, aspartame, sucralose, thaumatin, acesulfame potassium, stevioside, stevia extract, paramethoxy cinnamic aldehyde, neohesperidyl dihydrochalcone and perillartine. Typical levels are from 0.005 to 5 wt%, more preferably from 0.01 to 1 wt%.

### Other ingredients

Preferred toothpastes can include one or more bleaching agents such as peroxy compounds e.g. potassium peroxydiphosphate, effervescing systems such as sodium bicarbonate/citric acid systems, colour change systems, and chelating agents from 0.001 to 6 wt%, preferably at from 0.5 to 4 wt%, which include alkali metal salts of citric acid, alanine, glycine and serine.

The most preferred are the alkali metal salts of citric acid, especially potassium citrate and most preferably tri-potassium citrate.

Liposomes can be used to improve delivery or stability of one or more active ingredients. Preferred compositions may also include one or more of breath strips, sparkles, large silica particles, granules, beads, and flavour encapsulates for enhanced sensory benefits or for visual appeal.

Titanium dioxide can also be added to the composition for opacity. Titanium dioxide is generally included from 0.25 to 5 wt%.

It is preferred that the compositions contain a flavour. Suitable flavoring components include oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, ethyl vanillin, heliotropine, 4-cis-heptenal, diacetyl, methyl-para-tert-butyl phenyl acetate, and mixtures thereof. Coolants may also be part of the flavor system. Preferred coolants are the paramenthane carboxyamide agents such as N-ethyl-p-menthan-3-carboxamide (known commercially as "WS-3®") and mixtures thereof. The flavour is generally from 0.001 to 5 wt%.

The invention will now be further described with reference to the following nonlimiting examples.

### EXAMPLES

### Example-1: Preparation of preferred toothpastes for the experiments

Two preferred compositions were prepared for further experiments. The compositions are described in table 1.

**Table 1**

| **Ingredient/wt%** | **Composition 1** | **Composition 2** |
|---|---|---|
| calcium carbonate | 45.0 | 40.0 |
| sorbitol (70%) | 15.0 | 15.0 |
| Veegum™ HV | 0.8 | - |
| antibacterial particle* | 1.0 | 1.0 |
| sodium carboxymethyl cellulose | 0.5 | 0.5 |
| thickening silica | - | 2.0 |
| sodium lauryl sulphate (SLS) | 2.5 | 2.5 |
| sodium silicate | 1.7 | 1.7 |
| water and minors | to 100 | to 100 |

| | | |
|---|---|---|
| Note*: the particle had 1% cetyl pyridinuim chloride on kaolinite making its effective amount 0.01 wt% on the basis of complete formulation. The D50 of the particles was 0.5 µm. | | |

### Example 2: Single species re-growth assay with E.cloacae

Single species re-growth assay is an in-vitro assay which is conducted y to determine re-growth of bacteria. This assay can be used as a rapid method to predict differences in antibacterial activity between various actives in toothpastes. The test organism (*Enterobacter cloacae*) is resistant to SLS and hence is used to assess the efficacy of actives in formulations containing SLS. It involves monitoring the re-growth (by measuring absorbance at 630 nm) of a layer of bacteria after treatment with toothpaste slurry, followed by rinsing. Using a 96 well plate-reader, kinetic readings at every 30 minutes are taken for 24 hours at 630 nm. The time taken to reach a defined optical density (0.5) is calculated accordingly. The higher the time taken to reach this OD, the more efficacious is the antibacterial agent. Therefore the results can be directly related to efficacy of the antibacterial agent. In summary, the procedure involves treating the bacterial culture with the product and to monitor the growth (re-growth) of the bacterial culture by studying the changes in absorbance (optical density).

More absorbance means more re-growth. At the same time, an efficacious composition will delay the re-growth as much as possible.

In this particular test, the toothpaste slurries were prepared at 1 in 10 dilution and mixed thoroughly before performing the test. The 96 well plate was prepared with the bacterial layer and using a multi-channel pipette the test solutions were added and the wells were rinsed after two minutes contact time. The rinsing step was repeated twice and BHI (Brain Heart Infusion) media covered with a plate sealer and kept in a plate reader at 37 °C to assess the time taken for the re-growth.

The data is shown in table 2.

**Table 2**

| Composition | time taken/hours |
|---|---|
| Control (water alone) | 8.5 |
| Control 1 (Composition 1 without the particle) | >12.5 |
| Control 2 (Composition 2 without the particle) | >12.0 |
| Composition 1 | >48 |
| Composition 2 | >48 |
| Marketed popular toothpaste containing 0.3 % triclosan | >48 |

The results indicate that both the preferred compositions of table 1 provide significantly longer lasting protection against bacterial re-growth. The protection is comparable to that provided by the popular toothpaste containing 0.3 % triclosan. However, while each of the preferred compositions Composition 1 and Composition 2 have only an effective amount of 0.01 wt%, the marketed composition has 0.3 % triclosan.

### Example 3: Suspension test

This test is adapted from BS EN 1040 (modified with specific contact time). The efficacy of CPC-clay was tested in a 1 minute contact time. The test organism used was *E.cloacae* (*Enterobacter cloacae*).

The test culture was prepared by streaking out frozen stocks onto TSA (Tryptic Soy Agar) plate and incubating for 24 hours at 37 °C. A few colonies were then transferred onto a fresh TSA plate and incubated overnight at 37 °C. The colonies re-suspended in 0.8 % saline and adjusted to an OD of 0.8 at 620 nm, which corresponds to a count of 1x10⁸ cfu/ml. Test solutions were prepared by weighing the test material and by suspending it in MQ water as per the required concentration and vortex mixed thoroughly before performing the test. To 9 ml of the test solution added 1 ml of the test culture and after the specified contact time (1 minute), 1 ml of the this mixture was immediately neutralized in 9 ml D/E broth in duplicates. In the case of the control, 1 ml of test culture was added to 9 ml of saline and was serially diluted and plated on TSA. After solidification, the plates were incubated at 37°C for 48 hours and the residual colonies were counted.

**Table 3**

| **Composition** | **residual cfu/ml** | **residual log/ml** | **log reduction** |
|---|---|---|---|
| Water | 2.2X10⁷ | 7.3 | 0 |
| Composition 3^{#} (0.3 wt% particle) | 0 | 0 | > 5 log |
| Composition 4^{#} (0.8 wt% particle) | 0 | 0 | > 5 log |
| Marketed popular toothpaste containing 0.3wt% triclosan | 1.2X10⁵ | 5.1 | 2.2 |
| Marketed popular toothpaste containing 0.1 wt% triclosan | 1.8X10⁵ | 5.3 | 2.0 |
| Composition 3 having (0.3% triclosan + 0.2% zinc citrate)* | 1.2X10⁵ | 5.1 | 2.2 |
| Composition 3 having 0.2% zinc citrate* | 2.2X10⁷ | 7.3 | 0 |

| | | | |
|---|---|---|---|
| Note: ^{#} The balance of formulation was same as that of Composition 1. The only change is in the amount of the antibacterial particle. * These compositions are devoid of antibacterial particle. | | | |

The data in table 3 indicates how the preferred compositions (3 and 4) are superior to each of the other comparative compositions in terms of residual cfu/ml as well as the log reduction.

### Example 4: Salivary sediment quench assay

Salivary sediment quench assay involves human volunteers and the test is performed on the saliva collected from the volunteers. Objective of this test is to ascertain immediate effect of the antibacterial agent (in the toothpaste composition) on a mixed culture of bacteria which present in the pooled saliva. Since this assay utilise human saliva, it is considered to be very close to in-vivo/clinical studies.

Saliva was collected from volunteers, pooled, washed and standardized to get salivary sediment (2.0 OD).

This salivary sediment was added to the (1:5 diluted) toothpaste slurries (1:1). After 1 minute contact time, quench (D/E neutralising solution) was added to stop the activity of the test solution. Resulting suspension was serially diluted, plated and incubated anaerobically for seven days. The number of surviving colonies was then counted.

The results are indicated in the form of average residual log value of the bacteria present in the samples. Lower the value, better the product.

**Table 4**

| **Composition** | **Residual log cfu/ml** |
|---|---|
| Control (water) | 7.8 |
| Composition 1 | 4.2 |
| Composition 2 | 4.4 |
| Marketed popular toothpaste containing 0.3 % triclosan | 4.1 |

The data in table 4 indicates that not only are the preferred compositions 1 and 2 highly effective at controlling bacterial re-growth, but also they are equally good at providing immediate control on the bacterial count. As in the case of the explanation of data in table 2, this technical effect also is seen with only 0.01 wt% antibacterial agent whereas the marketed composition has 0.3 % triclosan.

## Claims

1. An toothpaste composition comprising:
(i) 5 to 60 wt% abrasive wherein said abrasive is a calcium containing abrasive;
(ii) an anionic surfactant; and,
(iii) a bipolar antimicrobial particle whose precursor is an asymmetric 1:1 or 2:1:1 clay particle comprising alternating tetrahedral and octahedral sheets terminating with a tetrahedral sheet at one external surface plane and an octahedral sheet at another external surface plane with an antibacterial agent attached to the coordinating cation on one of the said external surface plane where the antibacterial agent is a quaternary ammonium material.

2. A composition as claimed in claim 1 wherein in said antimicrobial particle, said antibacterial agent is attached to coordinating cations on the external surface of the octahedral surface plane.

3. A composition as claimed in claim 1 or 2 wherein ratio of clay to antimicrobial group is from 1:0.001 to 1:1.

4. A composition as claimed in any one of the preceding claims comprising 0.1 to 10 wt% bipolar antimicrobial particle.

5. A composition as claimed in any one of the preceding claims wherein said particle contains 1 % to 60 % loading of said antimicrobial agent.

6. A composition as claimed in any one of the preceding claims wherein median diameter of said particles (D50) is 0.1 to 10 µm.

7. A composition as claimed in any one of the preceding claims wherein said antibacterial agent is selected from a quaternary ammonium material comprising a single alkyl or alkenyl long chain having an average chain length greater than or equal to C₂₀ or a quaternary ammonium material selected from cetylpyridinium chloride (CPC), cetyltrimethylammonium chloride (CTAC), cetyltrimethylammonium bromide (CTAB), benzalkonium chloride (BKC), benzethonium chloride, cetrimide, quaternium, tetrabutyl ammonium bromide, undecylenamido propyltrimonium methosulphate, methylbenzethonium chloride, cetethyldimonium bromide, cetromonium tosylate, cocotrimonium chloride, dodecylbenzyltrimonium chloride, lauryl isoquinolium bromide, laurylpyridinium chloride, dequalinium chloride, domiphen bromide.

8. A composition as claimed in claim 7 wherein said quaternary ammonium material is selected from cetylpyridinium chloride (CPC), cetyltrimethylammonium chloride (CTAC), cetyltrimethylammonium bromide (CTAB), benzalkonium chloride (BKC), benzethonium chloride, cetrimide or quaternium.

9. A composition as claimed in any one of the preceding claims comprising 3.5 to 40 wt% humectants.

10. A composition as claimed in any one of the preceding claims further comprising 0.2 to 3 wt% of a smectite clay.

11. A composition as claimed in claim 10 wherein said smectite clay is an aluminium magnesium silicate clay.

12. A composition as claimed in any one of the preceding claims comprising up to 2 wt% thickening silica.

13. A method of promoting oral hygiene comprising a step of applying to the teeth an effective amount of a composition as claimed in claim 1.

14. A toothpaste composition as claimed in claim 1 for use for prolonged protection against bacteria present in the oral cavity.

## Patentansprüche

1. Zahnpasta-Zusammensetzung, umfassend:
(i) 5 bis 60 Gew.-% Schleifmittel, wobei das Schleifmittel ein Calcium enthaltendes Schleifmittel ist;
(ii) ein anionisches Tensid; und
(iii) ein bipolares antimikrobielles Teilchen, dessen Vorstufe ein asymmetrisches 1:1- oder 2:1:1-Tonteilchen ist, umfassend alternierende tetraedrische und oktaedrische Schichten, endend mit einer tetraedrischen Schicht an einer äußeren Oberflächenebene und einer oktaedrischen Schicht an einer anderen äußeren Oberflächenebene, mit einem antibakteriellen Mittel, das an dem koordinierenden Kation von einer der äußeren Oberflächenebenen gebunden ist, wobei das antibakterielle Mittel ein quaternäres Ammoniummaterial ist.

2. Zusammensetzung wie in Anspruch 1 beansprucht, wobei in dem antimikrobiellen Teilchen das antibakterielle Mittel an koordinierende Kationen auf der äußeren Oberfläche der oktaedrischen Oberflächenebene gebunden ist.

3. Zusammensetzung wie in Anspruch 1 oder 2 beansprucht, wobei das Verhältnis von Ton zu antimikrobieller Gruppe 1:0,001 bis 1:1 beträgt.

4. Zusammensetzung wie irgendeinem der vorhergehenden Ansprüche beansprucht, umfassend 0,1 bis 10 Gew.-% bipolare antimikrobielle Teilchen.

5. Zusammensetzung wie in irgendeinem der vorhergehenden Ansprüche beansprucht, wobei das Teilchen 1% bis 60% Beladung des antimikrobiellen Mittels enthält.

6. Zusammensetzung wie in irgendeinem der vorhergehenden Ansprüche beansprucht, wobei der Mediandurchmesser der Teilchen (D50) 0,1 bis 10 µm beträgt.

7. Zusammensetzung wie in irgendeinem der vorhergehenden Ansprüche beansprucht, wobei das antibakterielle Mittel ausgewählt ist aus einem quaternären Ammoniummaterial, das eine einzelne Alkyl- oder Alkenyl-Langkette mit einer durchschnittlichen Kettenlänge größer oder gleich C₂₀ umfasst, oder einem quaternären Ammoniummaterial ausgewählt aus Cetylpyridiniumchlorid (CPC), Cetyltrimethylammoniumchlorid (CTAC), Cetyltrimethylammoniumbromid (CTAB), Benzalkoniumchlorid (BKC), Benzethoniumchlorid, Cetrimid, Quaternium, Tetrabutylammoniumbromid, Undecylenamidopropyltrimoniummethosulfat, Methylbenzethoniumchlorid, Cetethyldimoniumbromid, Cetromoniumtosylat, Cocotrimoniumchlorid, Dodecylbenzyltrimoniumchlorid, Laurylisochinoliumbromid, Laurylpyridiniumchlorid, Dequaliniumchlorid, Domiphenbromid.

8. Zusammensetzung wie in Anspruch 7 beansprucht, wobei das quaternäre Ammoniummaterial ausgewählt ist aus Cetylpyridiniumchlorid (CPC), Cetyltrimethylammoniumchlorid (CTAC), Cetyltrimethylammoniumbromid (CTAB), Benzalkoniumchlorid (BKC), Benzethoniumchlorid, Cetrimid oder Quaternium.

9. Zusammensetzung wie in irgendeinem der vorhergehenden Ansprüche beansprucht, umfassend 3,5 bis 40 Gew.-% Feuchthaltemittel.

10. Zusammensetzung wie in irgendeinem der vorhergehenden Ansprüche beansprucht, ferner umfassend 0,2 bis 3 Gew.-% eines Smektit-Tons.

11. Zusammensetzung wie in Anspruch 10 beansprucht, wobei der Smektit-Ton ein Aluminiummagnesiumsilicat-Ton ist.

12. Zusammensetzung wie in irgendeinem der vorhergehenden Ansprüche beansprucht, umfassend bis zu 2 Gew.-% verdickendes Siliciumdioxid.

13. Verfahren zur Förderung der Mundhygiene, umfassend einen Schritt des Aufbringens einer wirksamen Menge einer Zusammensetzung wie in Anspruch 1 beansprucht auf die Zähne.

14. Zahnpasta-Zusammensetzung wie in Anspruch 1 beansprucht zur Verwendung für verlängerten Schutz gegen Bakterien, die in der Mundhöhle vorhanden sind.

## Revendications

1. Composition de dentifrice comprenant :
(i) de 5 à 60 % en masse d'abrasif dans laquelle ledit abrasif est un abrasif contenant du calcium ;
(ii) un tensioactif anionique ; et,
(iii) une particule antimicrobienne bipolaire dont le précurseur est une particule d'argile 1:1 ou 2:1:1 asymétrique comprenant des feuilles tétraédriques et octaédriques alternées se terminant avec une feuille tétraédrique sur un plan de surface externe et une feuille octaédrique sur un autre plan de surface externe avec un agent antibactérien fixé aux cations de coordination sur un dudit plan de surface externe où l'agent antibactérien est un matériau d'ammonium quaternaire.

2. Composition selon la revendication 1, dans laquelle dans ladite particule antimicrobienne, ledit agent antibactérien est fixé aux cations de coordination sur la surface externe du plan de surface octaédrique.

3. Composition selon la revendication 1 ou 2, dans laquelle le rapport d'argile à groupe antimicrobien est de 1:0,001 à 1:1.

4. Composition selon l'une quelconque des revendications précédentes comprenant de 0,1 à 10 % en masse de particules antimicrobiennes bipolaires.

5. Composition selon l'une quelconque des revendications précédentes, dans laquelle ladite particule contient de 1 % à 60 % de charge dudit agent antimicrobien.

6. Composition selon l'une quelconque des revendications précédentes, dans laquelle le diamètre médian desdites particules (D50) est de 0,1 à 10 µm.

7. Composition selon l'une quelconque des revendications précédentes, dans laquelle ledit agent antibactérien est choisi parmi un matériau d'ammonium quaternaire comprenant une seule chaîne longue alkyle ou alcényle présentant une longueur moyenne de chaîne supérieure ou égale à C₂₀ ou un matériau d'ammonium quaternaire choisi parmi le chlorure de cétylpyridinium (CPC), chlorure de cétyltriméthylammonium (CTAC), bromure de cétyltriméthylammonium (CTAB), chlorure de benzalkonium (BKC), chlorure de benzéthonium, cétrimide, quaternium, bromure de tétrabutylammonium, méthosulfate d'undécylènamido-propyltrimonium, chlorure de méthylbenzéthonium, bromure de cététhyldimonium, tosylate de cétromonium, chlorure de cocotrimonium, chlorure de dodécylbenzyltrimonium, bromure de laurylisoquinolium, chlorure de laurylpyridinium, chlorure de déqualinium, bromure de domiphène.

8. Composition selon la revendication 7, dans laquelle ledit matériau d'ammonium quaternaire est choisi parmi le chlorure de cétylpyridinium (CPC), chlorure de cétyltriméthylammonium (CTAC), bromure de cétyltriméthylammonium (CTAB), chlorure de benzalkonium (BKC), chlorure de benzéthonium, cétrimide ou quaternium.

9. Composition selon l'une quelconque des revendications précédentes comprenant de 3,5 à 40 % en masse d'humectants.

10. Composition selon l'une quelconque des revendications précédentes comprenant de plus de 0,2 à 3 % en masse d'argile de smectite.

11. Composition selon la revendication 10, dans laquelle ladite argile de smectite est une argile de silicate d'aluminium magnésium.

12. Composition selon l'une quelconque des revendications précédentes comprenant jusqu'à 2 % en masse de silice épaississante.

13. Procédé de promotion de l'hygiène orale comprenant une étape d'application aux dents d'une quantité efficace d'une composition selon la revendication 1.

14. Composition de dentifrice selon la revendication 1 pour une utilisation pour une protection prolongée contre des bactéries présentes dans la cavité orale.
